# EUROPEAN PATENT SPECIFICATION

(11) **EP 3 595 623 B1**
(45) Date of publication and mention of the grant of the patent: **01.05.2024**
(21) Application number: 18718505.3
(22) Date of filing: 14.03.2018
(51) Int. Cl.: A61K 8/34, A61K 8/37, A61K 8/49, A61K 8/55, A61K 8/60, A61Q 19/02, A61K 8/86, A61K 31/498, A61P 15/10, A61K 8/06

(54) **TOPICAL FORMULATION FOR THE TREATMENT OF PIGMENTED SKIN**
TOPISCHE FORMULIERUNG ZUR BEHANDLUNG VON PIGMENTIERTER HAUT
FORMULATION TOPIQUE DESTINÉE AU TRAITEMENT DE PROBLÈMES DE PIGMENTATION DE LA PEAU

(30) Priority: 14.03.2017 US 201762470901 P
(43) Date of publication of application: 22.01.2020
(73) Proprietor: Atir Holding S.A., 1118 Luxembourg (LU)
(72) Inventor: LURYA, Leonid, 76217 Rehovot (IL); MUNBLIT, Izabella, 9412502 Jerusalem (IL); TSIPE, Karina, 7173987 ModiIn (IL)
(74) Representative: Cabinet Laurent & Charras
(86) International application number: PCT/IB2018/051706
(87) International publication number: WO 2018/167689

(56) References cited:
- WO-A1-2012/140642
- US-A1- 2015 111 897

## Description

### FIELD AND BACKGROUND OF THE INVENTION

The present invention, in some embodiments thereof, relates to the field of skin care, and more particularly, but not exclusively, to methods involving compositions comprising heterocyclic compounds for the treatment of pigmented skin.

Skin lightening products have become increasingly popular in the past few years. The main purpose of skin lightening products is to lighten or whiten the skin complexion (which is of particular interest in certain Asian populations) or to treat pigmentation disorders such as chloasma, freckles, pregnancy marks and age spots. Several types of skin lightening products are presently available.

Compounds described as skin lightening agents include hydroquinone and derivatives thereof, such as arbutin; kojic acid and derivatives thereof, such as kojic dipalmitate; azelaic acid; and flavonoids, such as aloesin, resveratrol and glabridin. Such compounds are generally administered topically in a lotion or gel, and are believed to act via inhibition of tyrosinase, which is involved in melanin synthesis [Gillbro & Olsson, Int J Cosmet Sci 2011, 33:210-221]. Additional topically administered compounds used as skin lightening agents include niacinamide, which has been reported to inhibit melanosome transfer; and retinoids such as tretinoin, and alpha hydroxy acids such as lactic acid and glycolic acid, which may remove superficial layers of skin cells where hyperpigmented cells can accumulate [Gillbro & Olsson, Int J Cosmet Sci 2011, 33:210-221].

Glutathione and trans-4-aminomethyl cyclohexane carboxylic acid have been marketed as systemic skin lightening agents, but evidence for their efficacy is lacking [Malathi & Thappa, Indian J Dermatol Venerol Leprol 2013, 79:842-846].

International Patent Application PCT/IB03/00134 (published as WO 03/063875) describes a use of cyclic nucleotide PDE5 (phosphodiesterase-5) inhibitors such as sildenafil for the reduction of or prevention of scarring and/or fibrosis in various tissues.

International Patent Application PCT/US2010/061054 (published as WO 2011/075655) describes a use of cyclic nucleotide phosphodiesterase inhibitors such as sildenafil for treating peripheral vascular disease, including Reynaud's syndrome.

International Patent Applications PCT/IL2007/000404 (published as WO 2007/110868), PCT/IL2007/001174 (published as WO2008/117269) and PCT/IL2011/050077 (published as WO 2012/140642) describe heterocyclic compounds which exhibit a dopamine receptor (e.g., D4 receptor) agonist activity and/or a PDE5 inhibitory activity, for use in the treatment of sexual disorders. International Patent Application PCT/IL2011/050077 (published as WO 2012/140642) further describes pharmaceutical compositions for transdermal composition, in the form of an emulsion, which comprise the heterocyclic compound, along with propylene glycol, propylene glycol monolaurate, phosphatidyl choline, lauroyl macrogolglycerides, macrogolglycerol stearate, caprylocaproyl macrogolglycerides, and an aqueous solution of carboxymethyl cellulose.

Additional background art includes International Patent Applications PCT/US2003/033400 (published as WO 2004/037183) and PCT/US2009/053040 (published as WO 2010/019450).

### SUMMARY OF THE INVENTION

The invention is as defined in claims 1 to 13.

According to an aspect of some embodiments of the invention, there is provided a method of improving the appearance of aging skin in a subject in need thereof, the method comprising topically administering a composition comprising a compound of the general Formula I: 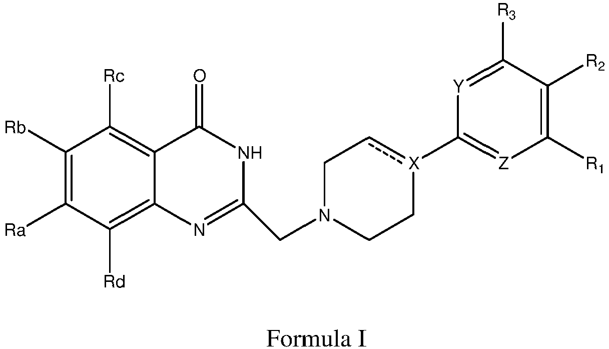
or a pharmaceutically acceptable salt thereof,
wherein:
   the dashed line denotes a saturated or non-saturated bond;
   X is selected from the group consisting of CH, C and N, such that when X is C the dashed line denotes a non-saturated bond and when X is CH or N the dashed line denotes a saturated bond;
   Y is N or CR₄;
   Z is N or CR₅; and
   Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
   and a carrier suitable for topical administration on skin, being in a form of a water-in-oil emulsion, the emulsion comprising:
      from 30 to 80 weight percents propylene glycol;
      from 7.5 to 30 weight percents phosphatidyl choline;
      from 5 to 20 weight percents lauroyl macrogolglycerides;
      from 2.5 to 10 weight percents macrogolglycerol stearate;
      from 0 to 2 weight percents ethanol;
      from 0 to 2 weight percents caprylocaproyl macrogolglycerides;
      from 0 to 2 weight percents propylene glycol monolaurate; and
      from 2 to 20 weight percents water.

According to an aspect of some embodiments of the invention, there is provided a method of lightening skin in a subject in need thereof, the method comprising topically administering a composition comprising a compound of the general Formula I: 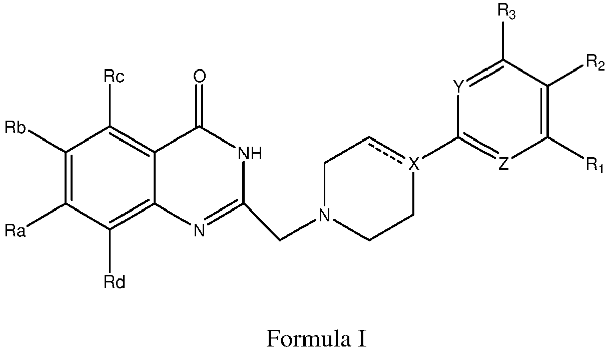
or a pharmaceutically acceptable salt thereof,
wherein:
   the dashed line denotes a saturated or non-saturated bond;
   X is selected from the group consisting of CH, C and N, such that when X is C the dashed line denotes a non-saturated bond and when X is CH or N the dashed line denotes a saturated bond;
   Y is N or CR₄;
   Z is N or CR₅; and
   Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
   and a carrier suitable for topical administration on skin, being in a form of a water-in-oil emulsion, said emulsion comprising:
      from 30 to 80 weight percents propylene glycol;
      from 7.5 to 30 weight percents phosphatidyl choline;
      from 5 to 20 weight percents lauroyl macrogolglycerides;
      from 2.5 to 10 weight percents macrogolglycerol stearate;
      from 0 to 2 weight percents ethanol;
      from 0 to 2 weight percents caprylocaproyl macrogolglycerides;
      from 0 to 2 weight percents propylene glycol monolaurate; and
      from 2 to 20 weight percents water.

According to an aspect of some embodiments of the invention, there is provided a method of treating uneven skin pigmentation in a subject in need thereof, the method comprising topically administering to the subject a composition comprising a compound of the general Formula I: 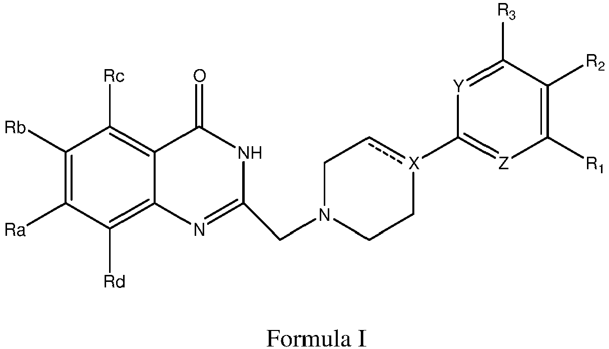
or a pharmaceutically acceptable salt thereof,
wherein:
   the dashed line denotes a saturated or non-saturated bond;
   X is selected from the group consisting of CH, C and N, such that when X is C the dashed line denotes a non-saturated bond and when X is CH or N the dashed line denotes a saturated bond;
   Y is N or CR₄;
   Z is N or CR₅; and
   Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
   and a carrier suitable for topical administration on skin, being in a form of a water-in-oil emulsion, said emulsion comprising:
      from 30 to 80 weight percents propylene glycol;
      from 7.5 to 30 weight percents phosphatidyl choline;
      from 5 to 20 weight percents lauroyl macrogolglycerides;
      from 2.5 to 10 weight percents macrogolglycerol stearate;
      from 0 to 2 weight percents ethanol;
      from 0 to 2 weight percents caprylocaproyl macrogolglycerides;
      from 0 to 2 weight percents propylene glycol monolaurate; and
      from 2 to 20 weight percents water.

According to some embodiments of the invention, a concentration of propylene glycol in the composition is about 60 weight percents.

According to some embodiments of the invention, a concentration of phosphatidyl choline in the composition is about 15 weight percents.

According to some embodiments of the invention, a concentration of lauroyl macrogolglycerides in the composition is about 10 weight percents.

According to some embodiments of the invention, a concentration of macrogolglycerol stearate in the composition is about 5 weight percents.

According to some embodiments of the invention, a concentration of ethanol in the composition is about 1 weight percent.

According to some embodiments of the invention, a concentration of caprylocaproyl macrogolglycerides in the composition is about 1 weight percent.

According to some embodiments of the invention, a concentration of propylene glycol monolaurate in the composition is about 1 weight percent.

According to some embodiments of the invention, a concentration of water in the composition is about 5.5 weight percents.

According to some embodiments of the invention, a concentration of the compound of general Formula I in the composition is at least 5 mg/ml.

According to some embodiments of the invention, the composition further comprises from 0.2 to 1 weight percent vitamin E TPGS (α-tocopheryl polyethylene glycol succinate).

According to some embodiments of the invention, a weight ratio of propylene glycol to phosphatidyl choline is in range of 3:1 to 5:1.

According to some embodiments of the invention, an aqueous phase of the emulsion comprises an aqueous solution of a carbomer.

According to some embodiments of the invention, a weight ratio of carbomer to water is in a range of from 1:500 to 1:100 (weight of carbomer: weight of water).

According to some embodiments of the invention, at least one of R₁-R₅ is selected from the group consisting of hydroxy and a moiety having the general Formula II: 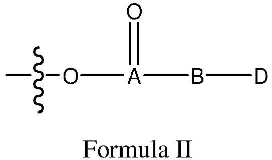 wherein:
A is selected from the group consisting of C and S=O;
B is absent or is a substituted or non-substituted, saturated or non-saturated alkylene chain; and
D is selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted.

According to some embodiments of the invention, R₁ is selected from the group consisting of hydroxy and the moiety having the general Formula II.

According to some embodiments of the invention, A is a carbon atom.

According to some embodiments of the invention, R₂-R₅ are each hydrogen.

According to some embodiments of the invention, Ra-Rd are each hydrogen.

According to some embodiments of the invention, X is N.

According to some embodiments of the invention, Y is CR₄ and Z is CR₅.

According to some embodiments of the invention, the compound is 2-((4-(3-hydroxyphenyl)piperazin-1-yl)methyl)quinazolin-4-one:

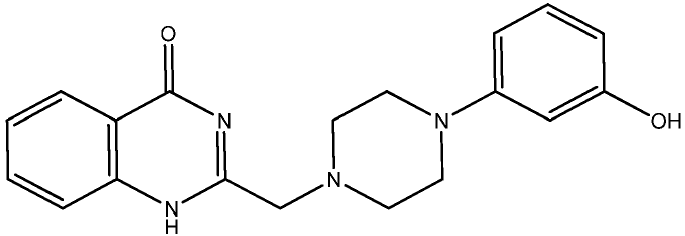

According to some embodiments of the invention, the composition is packaged in a packaging material and identified in print, in or on the packaging material, for use in improving the appearance of aging skin.

According to some embodiments of the invention, the composition is for use in a skin treatment for improving the appearance of aging skin.

According to some embodiments of the invention, the composition is for use in lightening skin.

According to some embodiments of the invention, the composition is for use in the treatment of uneven skin pigmentation.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### DESCRIPTION OF SPECIFIC EMBODIMENTS OF THE INVENTION

The present invention relates to the field of skin care, and more particularly, but not exclusively, to methods involving compositions comprising heterocyclic compounds for the treatment of pigmented skin.

Before explaining at least one embodiment of the invention in detail, it is to be understood that the invention is not limited in its application to the details set forth in the following description or exemplified by the Examples. Also, it is to be understood that the phraseology and terminology employed herein is for the purpose of description and should not be regarded as limiting.

While further studying the heterocyclic compounds and transdermal compositions described in International Patent Application PCT/IL2011/050077 (published as WO 2012/140642), the present inventors have serendipitously uncovered that compositions comprising such heterocyclic compounds can surprisingly reduce skin pigmentation and improve the appearance of skin.

The effect on skin was particularly surprising in that it was not limited to areas of the skin which were directly contacted with the compositions, unlike the effect of many compositions for treating skin.

According to an aspect of the present invention there is provided a method of improving the appearance of aging skin in a subject in need thereof, the method comprising topically administering a composition comprising a compound of the general Formula I: 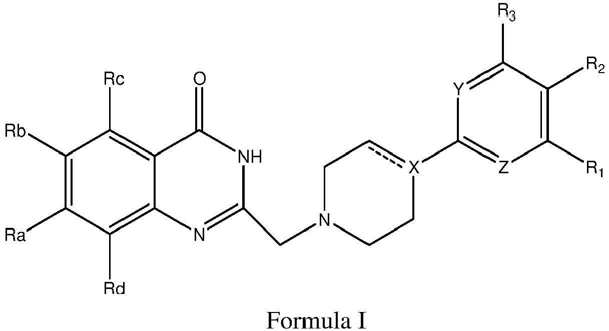 wherein X, Y, Z, Ra-Rd and R₁-R₅ are as defined herein (according to any of the respective embodiments described herein), and a carrier suitable for topical administration on skin. In some embodiments, the carrier is in a form of an emulsion. In exemplary embodiments, the carrier is in a form of a water-in-oil emulsion.

In some embodiments, of any of the embodiments described herein, the composition is for use in treating a medical and/or cosmetic condition in which topical administration of the compound of Formula I (according to any of the respective embodiments described herein) is beneficial.

In some embodiments, of any of the embodiments described herein, the composition is a cosmeceutical composition.

As used herein, the phrase "cosmeceutical composition" refers to a composition for topical administration as a cosmetic, which comprises a biologically active ingredient (e.g., the compound of Formula I, according to any of the respective embodiments described herein).

According to another aspect of the present invention there is provided a method of treating a cosmetic condition in which topical administration of the compound of Formula I (according to any of the respective embodiments described herein) is beneficial, the method comprising topically administering the composition described herein (according to any of the embodiments described herein).

In some embodiments, of any of the embodiments described herein, the composition is an anti-aging composition.

In some embodiments, of any of the embodiments described herein, the composition is for use in any one or more of a skin treatment for improving the appearance of aging skin; lightening skin; and/or treatment of uneven skin pigmentation.

According to another aspect of the present invention there is provided a method of lightening skin in a subject in need thereof, the method comprising topically administering the composition described herein (according to any of the embodiments described herein).

In some embodiments of any of the embodiments described herein relating to lightening skin, the method or use is for lightening a whole skin complexion.

In some embodiments of any of the embodiments described herein relating to lightening skin, the method or use is for lightening skin of a subject afflicted by uneven skin pigmentation, for example, skin comprising an uneven tone, melasma, skin discoloration, one or more acne marks and/or a scar.

According to another aspect of the present invention there is provided a method of treating uneven skin pigmentation in a subject in need thereof, the method comprising topically administering the composition described herein (according to any of the embodiments described herein).

In some embodiments of any one of the embodiments described herein, the uneven skin pigmentation is associated with aging, for example, uneven skin tone which appears after the age of 30, and/or age spots.

In some embodiments of any one of the embodiments described herein relating to uneven skin pigmentation, the uneven skin pigmentation is associated with hyperpigmentation of skin, for example, melasma, chloasma, lentigo (e.g., solar lentigo), and/or one or more dark spots such as age spots and/or freckles.

In some embodiments of any one of the embodiments described herein, the uneven skin pigmentation is associated with a lack of pigmentation, for example, vitiligo.

In such embodiments, lightening of the skin may beneficially reduce the contrast between pigmented regions and regions lacking pigmentation.

According to another aspect of the present invention there is provided a method of improving the appearance of aging skin in a subject in need thereof, the method comprising topically administering the composition described herein (according to any of the embodiments described herein).

In some embodiments, topical administration comprises contacting the skin with an amount of the composition which contains a therapeutically effective amount of the active ingredient of the composition (the compound of Formula I).

Herein, a "therapeutically effective amount" means an amount of one or more of the compounds of the present invention sufficiently effective to prevent, alleviate or ameliorate symptoms of a medical and/or cosmetic condition.

In some embodiments, topical administration of the compound effects intradermal and/or transdermal administration of the compound.

As used herein the terms "transdermal" and "transdermally" refer to administration of a compound across the skin of a subject for systemic distribution.

As used herein the terms "intradermal" and "intradermally" refer to administration of a compound into the skin of a subject, wherein the compound spreads through the skin, thereby reaching areas other than the area to which the compound was initially administered.

In some embodiments of any of the embodiments described herein, the subject is preferably a mammal, more preferably a human.

### Formulation of composition:

In some embodiments of any of the embodiments described herein, the composition is a water-in-oil emulsion.

As used herein and in the art, a "water-in-oil emulsion" is an emulsion characterized by an aqueous phase which is dispersed within a lipophilic phase. Any water-in-oil emulsion described herein may be, for example, in the form of a cream.

Herein, the term "emulsion" refers to a composition comprising liquids in two or more distinct phases (e.g., a hydrophilic phase and a lipophilic phase). Non-liquid substances (e.g., dispersed solids and/or gas bubbles) may optionally also be present.

In some such embodiments, the carrier is a water-in-oil emulsion and the compound of Formula I (according to any of the respective embodiments described herein) is predominantly (i.e., more than 50 %) in a lipophilic phase of the emulsion. In some embodiments, at least 60 % of the compound of Formula I is in the lipophilic phase of the emulsion. In some embodiments, at least 70 % of the compound of Formula I is in the lipophilic phase of the emulsion. In some embodiments, at least 80 % of the compound of Formula I is in the lipophilic phase of the emulsion. In some embodiments, at least 90 % of the compound of Formula I is in the lipophilic phase of the emulsion. In some embodiments, at least 95 % of the compound of Formula I is in the lipophilic phase of the emulsion. In some embodiments, at least 98 % of the compound of Formula I is in the lipophilic phase of the emulsion. In some embodiments, at least 99 % of the compound of Formula I is in the lipophilic phase of the emulsion.

In some embodiments of any of the embodiments described herein, the composition comprises at least one suitable solvent, for example, a solvent suitable for a lipophilic phase of an emulsion (according to any of the respective embodiments described herein).

Examples of suitable solvents include, without limitation, alkylene glycols (e.g., ethylene glycol, propylene glycol, butylene glycol) and diols (e.g., propane-1,3-diol), and fatty acid esters thereof (e.g., propylene glycol laurate, propylene glycol monolaurate).

In some embodiments of any of the embodiments described herein, the composition comprises at least one suitable solubilizing agent and/or sustained release agent, for example, a solubilizing agent and/or sustained release agent suitable for solubilizing and/or sustained release of an active compound (according to any of the respective embodiments described herein) in an emulsion (according to any of the respective embodiments described herein).

Examples of suitable solubilizing agents and/or sustained release agents include, without limitation, fatty acid esters (optionally derived from fatty acids of more than 10 carbon atoms in length) of glycerol and/or polyethylene glycol (macrogol). Lauroyl macrogolglycerides and stearoyl macrogolglycerides are exemplary fatty acid esters of glycerol and macrogol (optionally in a form of a mixture of glycerol and macrogol esters) suitable for use as a solubilizing agent and/or sustained release agent.

In some embodiments of any of the embodiments described herein, the composition comprises at least one suitable surfactant, for example, a surfactant suitable for a lipophilic phase of an emulsion (according to any of the respective embodiments described herein).

Examples of suitable surfactants include, without limitation, phospholipids (e.g., phosphatidyl choline, phosphatidyl ethanolamine, phosphatidyl serine, phosphatidyl inositol and phosphatidic acid) and fatty acid esters (optionally derived from fatty acids of up to 10 carbon atoms in length) of glycerol and/or polyethylene glycol (macrogol). Phosphatidyl choline is an exemplary phospholipid, and caprylocaproyl macrogolglycerides are exemplary fatty acid esters of glycerol and macrogol (optionally in a form of a mixture of glycerol and macrogol esters), suitable for use as surfactants.

In some embodiments of any of the embodiments described herein, a concentration of propylene glycol in the composition (e.g., emulsion) is in a range of from 30 to 80 weight percents. In some embodiments, the concentration of propylene glycol is in a range of from 40 to 80 weight percents. In some embodiments, the concentration of propylene glycol is in a range of from 50 to 80 weight percents. In some embodiments, the concentration of propylene glycol is in a range of from 55 to 80 weight percents. In some embodiments, the concentration of propylene glycol is in a range of from 60 to 80 weight percents.

In some embodiments of any of the embodiments described herein, a concentration of propylene glycol in the composition (e.g., emulsion) is in a range of from 40 to 75 weight percents. In some embodiments, the concentration of propylene glycol is in a range of from 50 to 70 weight percents. In some embodiments, the concentration of propylene glycol is in a range of from 55 to 65 weight percents. In some embodiments, the concentration of propylene glycol is about 60 weight percents.

In some embodiments of any of the embodiments described herein, a concentration of phosphatidyl choline in the composition (e.g., emulsion) is in a range of from 7.5 to 30 weight percents. In some embodiments, the concentration of phosphatidyl choline is in a range of from 10 to 30 weight percents. In some embodiments, the concentration of phosphatidyl choline is in a range of from 15 to 30 weight percents.

In some embodiments of any of the embodiments described herein, a concentration of phosphatidyl choline in the composition (e.g., emulsion) is in a range of from 10 to 20 weight percents. In some embodiments, the concentration of propylene glycol is about 15 weight percents.

In some embodiments of any of the embodiments described herein, a weight ratio of propylene glycol to phosphatidyl choline is in a range of from 3:1 to 5:1 (propylene glycol : phosphatidyl choline). In some embodiments, the ratio is about 4:1. In some such embodiments, the concentration of propylene glycol in the composition is in a range of from 30 to 80 weight percents. In some embodiments, a concentration of propylene glycol in the composition is in a range of from 40 to 75 weight percents. In some embodiments, the concentration of propylene glycol is in a range of from 50 to 70 weight percents. In some embodiments, the concentration of propylene glycol is in a range of from 55 to 65 weight percents. In some embodiments, the concentration of propylene glycol is about 60 weight percents.

In some embodiments of any of the embodiments described herein, a weight ratio of propylene glycol to phosphatidyl choline is in a range of from 3:1 to 5:1 (propylene glycol: phosphatidyl choline), and a concentration of phosphatidyl choline in the composition is in a range of from 7.5 to 30 weight percents. In some embodiments, the ratio is about 4:1. In some embodiments, the concentration of phosphatidyl choline is in a range of from 10 to 20 weight percents. In some embodiments, the concentration of phosphatidyl choline is about 15 weight percents.

In some embodiments of any of the embodiments described herein, a concentration of lauroyl macrogolglycerides in the composition (e.g., emulsion) is in a range of from 5 to 20 weight percents. In some embodiments, the concentration of lauroyl macrogolglycerides is in a range of from 7.5 to 15 weight percents. In some embodiments, the concentration of lauroyl macrogolglycerides is about 10 weight percents.

In some embodiments of any of the embodiments described relating to lauroyl macrogolglycerides, the lauroyl macrogolglycerides comprise lauroyl polyoxyl-32 glycerides. Suitable lauroyl macrogolglycerides are available, for example, as Gelucire^{®} 44/14.

In some embodiments of any of the embodiments described herein, a concentration of macrogolglycerol stearate in the composition (e.g., emulsion) is in a range of from 2.5 to 10 weight percents. In some embodiments, the concentration of macrogolglycerol stearate is in a range of from 4 to 6 weight percents. In some embodiments, the concentration of macrogolglycerol stearate is about 5 weight percents. Suitable macrogolglycerol stearate is available, for example, as Gelucire^{®} 50/13.

In some embodiments of any of the embodiments described herein, a concentration of ethanol in the composition (e.g., emulsion) is at least 0.1 weight percent. In some embodiments, the concentration of ethanol is at least 0.2 weight percent. In some embodiments, the concentration of ethanol is at least 0.5 weight percent. In some embodiments, the concentration of ethanol is at least 1 weight percent.

In some embodiments of any of the embodiments described herein, a concentration of ethanol in the composition (e.g., emulsion) is in a range of from 0 to 2 weight percents. In some embodiments, the concentration of ethanol is in a range of from 0.1 to 2 weight percents. In some embodiments, the concentration of ethanol is in a range of from 0.2 to 2 weight percents. In some embodiments, the concentration of ethanol is in a range of from 0.5 to 2 weight percents. In some embodiments, the concentration of ethanol is in a range of from 1 to 2 weight percents.

In some embodiments of any of the embodiments described herein, a concentration of ethanol in the composition (e.g., emulsion) is in a range of from 0.5 to 1.5 weight percent. In some embodiments, the concentration of ethanol is about 1 weight percent.

In some embodiments of any of the embodiments described herein, a concentration of caprylocaproyl macrogolglycerides in the composition (e.g., emulsion) is in a range of from 0 to 2 weight percents. In some embodiments, the concentration of caprylocaproyl macrogolglycerides is in a range of from 0.1 to 2 weight percents. In some embodiments, the concentration of caprylocaproyl macrogolglycerides is in a range of from 0.2 to 2 weight percents. In some embodiments, the concentration of caprylocaproyl macrogolglycerides in a range of from 0.5 to 2 weight percents. In some embodiments, the concentration of caprylocaproyl macrogolglycerides is in a range of from 1 to 2 weight percents.

In some embodiments of any of the embodiments described herein, a concentration of caprylocaproyl macrogolglycerides in the composition (e.g., emulsion) is in a range of from 0.5 to 1.5 weight percent. In some embodiments, the concentration of caprylocaproyl macrogolglycerides is about 1 weight percent.

In some embodiments of any of the embodiments described relating to caprylocaproyl macrogolglycerides, the caprylocaproyl macrogolglycerides comprise caprylocaproyl polyoxyl-8 glycerides. Suitable caprylocaproyl macrogolglycerides are available, for example, as Labrasol^{®}.

In some embodiments of any of the embodiments described herein, a concentration of propylene glycol monolaurate in the composition (e.g., emulsion) is in a range of from 0 to 2 weight percents. In some embodiments, the concentration of propylene glycol monolaurate is in a range of from 0.1 to 2 weight percents. In some embodiments, the concentration of propylene glycol monolaurate is in a range of from 0.2 to 2 weight percents. In some embodiments, the concentration of propylene glycol monolaurate in a range of from 0.5 to 2 weight percents. In some embodiments, the concentration of propylene glycol monolaurate is in a range of from 1 to 2 weight percents.

In some embodiments of any of the embodiments described herein, a concentration of propylene glycol monolaurate in the composition (e.g., emulsion) is in a range of from 0.5 to 1.5 weight percent. In some embodiments, the concentration of propylene glycol monolaurate is about 1 weight percent. Suitable propylene glycol monolaurate is available, for example, as Lauroglycol^{™} 90.

In some embodiments of any of the embodiments described herein, a concentration of water in the carrier is in a range of 2 to 20 weight percents. In some embodiments, a concentration of water in the carrier is in a range of 2 to 15 weight percents. In some embodiments, a concentration of water in the carrier is in a range of 2 to 10 weight percents. In some embodiments, a concentration of water in the carrier is in a range of 3 to 8 weight percents. In exemplary embodiments, a concentration of water in the carrier is about 5.5 weight percents.

In some embodiments of any of the embodiments described herein, the composition further comprises a gel-forming agent. In some embodiments, the gel-forming agent is a hydrophilic polymer, optionally a water-soluble polymer.

In some embodiments of any of the embodiments described herein, the gel-forming agent (e.g., a hydrophilic gel-forming agent) reduces a rate of coalescence of aqueous droplets in a water-in-oil emulsion (optionally a water-in-oil emulsion according to any of the respective embodiments described herein), in comparison with aqueous droplets in a an emulsion differing only by the absence of the gel-forming agent.

In some embodiments, the gel-forming agent is a crosslinked polyacrylic acid, such as a carbomer, e.g., carboxypolyalkylenes that may be obtained commercially under the trademark Carbopol^{®}. Carbopol^{®} Ultrez 10 cross-linked polyacrylic acid is an example of a particularly suitable carbomer.

Other types of optional polymers in this context are hydrophilic polymers such as polyethylene oxides, polyoxyethylene-polyoxypropylene copolymers and polyvinylalcohol; cellulosic polymers such as hydroxypropyl cellulose, hydroxyethyl cellulose, hydroxypropyl methylcellulose, hydroxypropyl methylcellulose phthalate, and methyl cellulose; gums such as tragacanth and xanthan gum; sodium alginate; and gelatin.

In some embodiments of any of the embodiments described herein, the composition is an emulsion wherein an aqueous phase of the emulsion comprises a gel-forming agent (e.g., carbomer) in solution according to any of the respective embodiments described herein, that is, the aqueous phase comprises an aqueous solution of the gel-forming agent. In some embodiments, a weight ratio of carbomer to water in the composition is in a range of from 1:500 to 1:100. In some embodiments, a weight ratio of carbomer to water is in a range of from 1:350 to 1:150. In some embodiments, a weight ratio of carbomer to water is about 1:250.

According to some embodiments of any of the embodiments described herein, the carrier comprises water, propylene glycol, phosphatidyl choline, lauroyl macrogolglycerides and macrogolglycerol stearate, each of the aforementioned ingredients being in a concentration according to any of the respective embodiments described herein. In some embodiments, the carrier further comprises ethanol, caprylocaproyl macrogolglycerides and/or propylene glycol monolaurate, each of the aforementioned ingredients being in a concentration according to any of the respective embodiments described herein. In some such embodiments, the carrier comprises ethanol, and optionally further comprises caprylocaproyl macrogolglycerides and/or propylene glycol monolaurate. In some embodiments, the carrier further comprises a carbomer in a concentration according to any of the respective embodiments described herein.

According to optional embodiments, the composition further comprises vitamin E TPGS (α-tocopheryl polyethylene glycol succinate), for example, at a concentration of up to 1 weight percent of the composition. In some embodiments, the concentration of vitamin E TPGS is in a range of from 0.2 to 1 weight percent. In some embodiments, the concentration of vitamin E TPGS is about 0.5 weight percent.

As exemplified herein, compositions formulated for topical administration as described herein may advantageously comprise a relatively high concentration of a compound having Formula I, for example, a concentration of at least 5 mg/ml, optionally from 5 to 20 mg/ml, and optionally from 5 to 15 mg/ml. In some embodiments, the concentration of the compound having Formula I is at least 7.5 mg/ml, optionally from 7.5 to 20 mg/ml, and optionally from 7.5 to 15 mg/ml. In some embodiments, the concentration of the compound having Formula I is at least 10 mg/ml, optionally from 10 to 20 mg/ml, and optionally from 10 to 15 mg/ml. In some embodiments, the concentration of the compound having Formula I is about 10 mg/ml.

Herein, the phrase "carrier suitable for topical administration" refers to a carrier that does not cause significant irritation to an organism upon topical administration and does not abrogate the biological activity and properties of the administered compound.

In some embodiments of any of the embodiments described herein, the composition is a cosmetic composition which alters the skin (as opposed, for example, to cosmetic compositions, e.g., make-up, which merely mask the skin).

Techniques for formulation and administration of drugs may be found in "Remington's Pharmaceutical Sciences" Mack Publishing Co., Easton, PA, latest edition.

Compositions described herein according to various embodiments of the present invention may be manufactured by processes well known in the art, e.g., by means of conventional mixing, dissolving, granulating, dragee-making, levigating, emulsifying, encapsulating, entrapping or lyophilizing processes.

In some embodiments of any of the embodiments described herein, the composition is a self-emulsifying composition.

Herein, the term "self-emulsifying composition" means that a mixture of ingredients other than water at the amounts present in the composition (e.g., propylene glycol, phosphatidyl choline, lauroyl macrogolglycerides, macrogolglycerol stearate, ethanol, caprylocaproyl macrogolglycerides, and propylene glycol monolaurate, in amounts indicated herein) undergo spontaneous emulsification upon contact with water, for example, to form an emulsion.

Self-emulsifying compositions can optionally be prepared by adding water or aqueous solution (e.g., an aqueous solution according to any of the embodiments described herein) to a pre-prepared mixture (optionally an isotropic mixture) of the other ingredients of the composition.

According to some embodiments of any of the embodiments described herein, the composition is formulated for topical administration, and optionally for intradermal and/or transdermal administration.

As exemplified in the Examples section below, compositions formulated for topical administration, such as are described herein, allow the compound to exhibit a desired activity (e.g., as described herein) over a large area of skin.

Without being bound by any particular theory, it is believed that topical administration provides gradual uptake into the skin and/or bloodstream over the course of a considerable time period, and can therefore provide a long lasting, therapeutically effective concentration of the compound in the body, even if the compound has a relatively short half-life in plasma.

A composition formulated for topical (e.g., intradermal and/or transdermal) administration may optionally be present in a patch, a swab, a pledget, and/or a pad.

Dermal patches and the like may comprise some or all of the following components: a composition (e.g., as described herein), a liner for protecting the patch during storage, which is optionally removed prior to use, an adhesive for adhering different components together and/or adhering the patch to the skin, a backing which protects the patch from the outer environment, and/or a membrane which controls release of a drug into the skin.

According to optional embodiments, the composition is stable (e.g., devoid of substantial chemical changes and/or phase separation) at room temperature (e.g., 20 °C) for at least 2 weeks, optionally at least 1 month, optionally at least 2 months, optionally at least 6 months, and optionally at least 1 year.

As described herein, topical compositions described herein may provide for a continuous release of the compound into the body of a subject. In some embodiments, the composition is characterized by an ability to release the compound (e.g., a compound according to Formula I) for at least 2 hours, optionally for at least 3 hours, optionally for at least 4 hours, and optionally for at least 6 hours, upon administration of the composition on a skin of a subject.

Release of a compound from an applied composition may be determined quantitatively by any suitable technique used in the art.

Optionally, the release is determined *in vivo,* by monitoring plasma concentrations of the compound. Using standard pharmacokinetic analysis, the absorption of a compound into the plasma may be determined for each point in time, based on the observed concentration of the compound in plasma and on the rate of clearance of the compound.

Alternatively, the release may be determined *in vitro,* by monitoring permeation of a compound through skin in a Franz diffusion cell.

Herein, a composition is considered to be able to release a compound for a particular period of time (e.g., at least two hours) if the rate at which the compound permeates the skin (e.g., absorption into plasma) during the period of time is at least half of the maximal rate achieved after administration of the composition.

Compositions suitable for use in context of embodiments of the present invention include compositions wherein the active ingredients are contained in a therapeutically effective amount for achieving the intended purpose.

Determination of a therapeutically effective amount is well within the capability of those skilled in the art, especially in light of the detailed disclosure provided herein.

The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p. 1).

Dosage amount and interval may optionally be adjusted individually to provide plasma levels of the active moiety which are sufficient to maintain desired effects, termed the minimal effective concentration (MEC). The MEC will vary for each preparation, but can be estimated from, e.g., the concentration necessary in plasma to achieve, over a given period of time, a desired effect in human subjects (e.g., in the skin of the subjects). Dosages necessary to achieve the MEC will depend on individual characteristics and route of administration. HPLC assays or bioassays can be used to determine plasma concentrations.

Dosage intervals can also be determined using the MEC value. Preparations should be administered using a regimen, which maintains plasma levels above the MEC for 10-90 % of the time, preferable between 30-90 % and most preferably 50-90 %.

The amount of a composition to be administered will, of course, be dependent on the subject being treated, the severity of the affliction, the manner of administration, the judgment of the prescribing physician, etc.

The amount of the compound to be administered can depend on the pharmacokinetics of the compound, for example, a half-life of the administered compound and/or a product of hydrolysis of the administered compound in plasma, and/or a rate of absorption of an administered compound when administered topically.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack. The pack or dispenser device may be accompanied by instructions for administration. The pack or dispenser may also be accompanied by a notice associated with the container in a form prescribed by a governmental agency regulating the manufacture, use or sale of cosmetics and/or pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions as described herein may also be prepared, placed in an appropriate container, and labeled for a use described herein (e.g., treating sexual disorder, the lightening of skin, treating uneven skin pigmentation and/or improving the appearance of aging skin) in a subject in need thereof.

Thus, according to some embodiments of the present invention, the compositions described herein are packaged in a packaging material and identified in print, in or on the packaging material, for a use described herein (e.g., the lightening of skin, treating uneven skin pigmentation and/or improving the appearance of aging skin) in a subject in need thereof.

### Compound structure:

According to any of the aspects of embodiments of the present invention, the compound has the general Formula I: 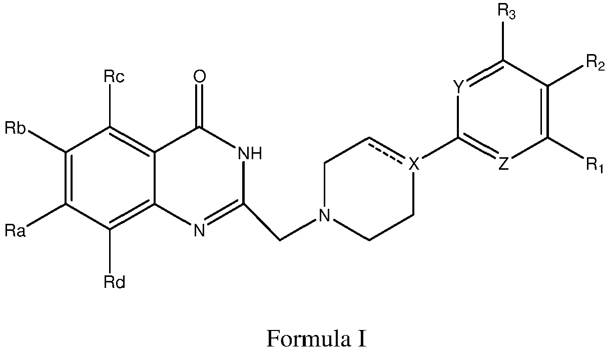 wherein:
the dashed line denotes a saturated or non-saturated bond;
X is CH, C or N such that when X is C the dashed line denotes a non-saturated bond and when X is CH or N the dashed line denotes a saturated bond;
Y is N or CR₄ (e.g., CR₄);
Z is N or CR₅ (e.g., CR₅); and
Ra-Rd, and R₁-R₅ are each independently hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and/or thiocarbamyl, each being substituted or non-substituted.

In some embodiments of any of the embodiments described herein, at least one of R₁-R₅ is hydroxy, a carboxy ester moiety or a sulfonate ester moiety, such that the aryl or heteroaryl ring comprising Y and Z is substituted by at least one hydroxy group, and/or at least one carboxy ester or sulfonate ester (which may be considered as derivatives of a hydroxy group). Carboxy and sulfonate ester moieties are described in more detail below. In some embodiments, R₁ is hydroxy, a carboxy ester or a sulfonate ester.

In some embodiments of any of the embodiments described herein, one of R₁-R₅ is hydroxy, a carboxy ester or a sulfonate ester, and the others are hydrogen. In some embodiments, R₁ is hydroxy, a carboxy ester or a sulfonate ester, and R₂-R₅ are hydrogen.

In some embodiments of any of the embodiments described herein, one or more of R₁-R₅ (optionally only one of R₁-R₅) is a substituent (i.e., a group other than hydrogen) other than an ester moiety described herein. In some embodiments, the one or more substituent(s) is alkyl, hydroxy, alkoxy, halide and/or nitrile.

In some embodiments of any of the embodiments described herein, at least one of R₁-R₅ is hydroxy. In some embodiments, R₁ is hydroxy.

In some embodiments of any of the embodiments described herein, one of R₁-R₅ is hydroxy, and the others are hydrogen. In some embodiments, R₁ is hydroxy, and R₂-R₅ are hydrogen.

In some embodiments of any of the embodiments described herein, X is N, and the dashed line denotes a saturated bond.

In some embodiments of any of the embodiments described herein, the compound comprises a phenyl ring, wherein Y is CR₄ and Z is CR₅.

According to some embodiments, the bicyclic quinazolin-4-one moiety in Formula I is a non-substituted bicyclic moiety, such that each of Ra-Rd is hydrogen.

Alternatively, at least one of Ra-Rd is other than hydrogen, such that the bicyclic moiety is substituted.

In some embodiments, at least one of Ra-Rd is alkyl, hydroxy, alkoxy and/or halide.

In some embodiments, Ra is hydrogen or halide, short alkyl (being 1-4 carbon atoms in length) or short alkoxy (being 1-4 carbon atoms in length). In some embodiments, Ra is hydrogen or halide. In some embodiments, the halide is chloride. In some embodiments, the alkyl is ethyl. In some embodiments, the alkoxy is methoxy.

In some embodiments, Rb is hydrogen, halide (e.g., chloride), short alkyl (being 1-4 carbon atoms in length, and optionally being an aryl-substituted alkyl, e.g., benzyl), or alkoxy (being 1-4 carbon atoms in length, e.g., methoxy). In some embodiments, the alkyl is ethyl, propyl, trifluoromethyl or benzyl. In some embodiments, Rb is hydrogen, halide or alkoxy. According to exemplary embodiments, Rb is hydrogen or halide. In some embodiments, the halide is chloride. In some embodiments, the alkoxy is methoxy.

In some embodiments, Rc is hydrogen, alkoxy (being 1-4 carbon atoms in length), halide or alkyl (being 1-4 carbon atoms in length). In some embodiments, Rc is hydrogen, halide or alkyl. In some embodiments, the alkyl is methyl. In some embodiments, the halide is fluoride.

In some embodiments, Rd is hydrogen or alkyl. In some embodiments, the alkyl is methyl or propyl.

According to exemplary embodiments, Ra, Rc and Rd are each hydrogen.

R-55 (2-((4-(3-hydroxyphenyl)piperazin-1-yl)methyl)quinazolin-4-one) is an exemplary compound suitable for use according to embodiments of the invention.

Additional examples of compounds having general Formula I, which are suitable for use according to embodiments of the invention, as well as processes for preparing such compounds, are presented in International Patent Application Publication WO 2012/140642.

Each of the compounds described herein can further be in a form of a pharmaceutically acceptable salt thereof.

As used herein, the phrase "pharmaceutically acceptable salt" refers to a charged species of the parent compound and its counter-ion, which is typically used to modify the solubility characteristics of the parent compound and/or to reduce any significant irritation to an organism by the parent compound, while not abrogating the biological activity and properties of the administered compound.

In the context of the present embodiments, a pharmaceutically acceptable salt of the compounds described herein may optionally be an acid addition salt comprising at least one amine group of the compound (e.g., an amine group in a piperazine moiety) which is in a form of an ammonium ion (e.g., a quaternary ammonium ion), in combination with at least one counter ion, derived from the selected acid, that forms a pharmaceutically acceptable salt.

Depending on the stoichiometric proportions between the base (the amine group(s)) and the acid in the salt, the acid additions salts can be either mono-addition salts or poly-addition salts.

The phrase "mono-addition salt", as used herein, refers to a salt in which the stoichiometric ratio between the acid anion and amine cation is 1:1, such that the acid addition salt includes one molar equivalent of the acid per one molar equivalent of the compound.

The phrase "poly-addition salt", as used herein, refers to a salt in which the stoichiometric ratio between the acid anion and the amine cation is greater than 1:1 and is, for example, 2:1, 3:1, 4:1 and so on, such that the acid addition salt includes two or more molar equivalents of the acid per one molar equivalent of the compound.

The acid addition salts of the compounds described herein are therefore complexes formed between one or more amino groups of the drug and one or more equivalents of an acid.

The acid addition salts may include a variety of organic and inorganic acids, such as, but not limited to, hydrochloric acid which affords a hydrochloric acid addition salt, hydrobromic acid which affords a hydrobromic acid addition salt, acetic acid which affords an acetic acid addition salt, ascorbic acid which affords an ascorbic acid addition salt, benzenesulfonic acid which affords a besylate addition salt, camphorsulfonic acid which affords a camphorsulfonic acid addition salt, citric acid which affords a citric acid addition salt, maleic acid which affords a maleic acid addition salt, malic acid which affords a malic acid addition salt, methanesulfonic acid which affords a methanesulfonic acid (mesylate) addition salt, naphthalenesulfonic acid which affords a naphthalenesulfonic acid addition salt, oxalic acid which affords an oxalic acid addition salt, phosphoric acid which affords a phosphoric acid addition salt, toluenesulfonic acid which affords a p-toluenesulfonic acid addition salt, succinic acid which affords a succinic acid addition salt, sulfuric acid which affords a sulfuric acid addition salt, tartaric acid which affords a tartaric acid addition salt and trifluoroacetic acid which affords a trifluoroacetic acid addition salt. Each of these acid addition salts can be either a mono-addition salt or a poly-addition salt, as these terms are defined hereinabove.

Further, each of the compounds described herein, including the salts thereof, can be in a form of a solvate or a hydrate thereof.

The term "solvate" refers to a complex of variable stoichiometry (e.g., di-, tri-, tetra-, penta-, hexa-, and so on), which is formed by a solute (the heterocyclic compounds described herein) and a solvent, whereby the solvent does not interfere with the biological activity of the solute.

The term "hydrate" refers to a solvate, as defined hereinabove, where the solvent is water.

The present embodiments further encompass any stereoisomers (enantiomers and diastereomers) of the compounds described herein, as well as any isomorph thereof.

### Esters:

In some embodiments of any one of the embodiments described herein, at least one of R₁-R₅ is an ester moiety, for example, a carboxy or sulfonate which is a carboxy ester or sulfonate ester, respectively. Such compounds are referred to herein also as "esterified compounds".

In some embodiments of any one of the embodiments described herein, the esterified compound is an esterified derivative of R-55, differing from R-55 only in that an ester moiety (according to any of the respective embodiments described herein) is present at the R₁ position instead of a hydroxy group. Additional examples of esterified compounds having general Formula I, which are suitable for use according to embodiments of the invention, are presented in International Patent Application Publication WO 2012/140642.

In some embodiments of any one of the embodiments described herein, the ester moiety has the general Formula II: 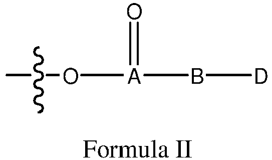 wherein:
A is selected from the group consisting of a carbon atom and S=O;
B is absent or is a substituted or non-substituted, saturated or non-saturated alkylene chain; and
D is selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted.

In some embodiments, D is alkyl. The alkyl is optionally non-substituted.

In some embodiments, D is alkenyl, for example, non-substituted alkenyl.

Without being bound by any particular theory, it is believed that the ester moiety of the esterified compound undergoes gradual hydrolysis *in vivo,* to release an active compound comprising a hydroxy group in place of the ester moiety. It is further believed that the esterified compound may provide a higher availability in plasma following topical administration.

As used herein, an "alkylene chain" refers to a bi-radical moiety (i.e., a divalent radical) comprising 1-20 carbon atoms covalently linked to one another by single, double or triple bonds. In a "saturated" alkylene chain, the carbon atoms are linked to one another solely by single bonds, whereas an "unsaturated" alkylene chain comprises at least one double bond and/or triple bond between carbon atoms. The alkylene chain is optionally substituted by one or more substituents, whereby the substituents can be, for example, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, nitrile, nitro, azide, phosphonyl, phosphinyl, oxo, carbonyl, thiocarbonyl, urea, thiourea, carbamyl, N-carbamyl, O-thiocarbamyl, N-thiocarbamyl, C-amido, N-amido, C-carboxy, O-carboxy, sulfonamido, and amino, as these terms are defined herein. Optionally, B is an alkylene chain of up to 10 carbon atoms, optionally of up to 4 carbon atoms (e.g., a saturated alkylene of 1 to 4 carbon atoms), and optionally 1 or 2 carbon atoms. Optionally, B is a saturated alkylene chain, and the saturated alkylene is optionally non-substituted (e.g., CH₂, CH₂CH₂). Alternatively, the saturated alkylene chain may be substituted. In some embodiments, the saturated alkylene chain is substituted by hydroxy.

As described in International Patent Application Publication WO 2012/140642, the rate of release of the active therapeutic agent (e.g., by hydrolysis of the esterified compound) can be controlled by selection of an appropriate ester moiety. Thus, for example, carboxy esters comprising small unsubstituted moieties (e.g., alkyl, cycloalkyl, aryl or heteroaryl) resulted in relatively rapid hydrolysis (e.g., wherein T_{1/2} in human plasma is about 150 minutes or less), whereas sulfonate ester moieties resulted in relatively slow hydrolysis (e.g., wherein the half-life (T_{1/2}) in human plasma is over 1000 minutes or less).

Thus, the half-life in human plasma may optionally be manipulated as desired by selecting a carboxy ester (for shorter half-lives) or a sulfonate ester (for longer half-lives).

Thus, in some embodiments, the ester is a sulfonate ester, for example, an alkyl-substituted sulfonate ester or an aryl-substituted sulfonate ester. Methanesulfonate is a non-limiting example of a suitable alkyl-substituted sulfonate ester moiety and p-toluenesulfonate is non-limiting example of a suitable aryl-substituted ester moiety.

In some embodiments of any one of the embodiments described herein, the ester moiety is carboxy (i.e. A is a carbon atom) rather than sulfonate (wherein A is S=O).

For some applications relating to skin, a very short (e.g., less than 10 minutes, less than 30 minutes, less than 60 minutes) T_{1/2} in human plasma at 37 °C (e.g., as is typical of small carboxy esters such as propionate ester, wherein B is absent and D is ethyl) is undesirable.

Without being bound by any particular theory, it is believed that an active agent comprising a hydroxy group (e.g., formed upon hydrolysis of an esterified compound described herein) is more susceptible to partial or complete inactivation and/or clearance from the body, due to metabolic processes *in vivo* (e.g., by glucuronidation and/or sulfation of the hydroxy group). It is further believed that rapid hydrolysis may result in considerable inactivation and/or clearance from the body before the active agent reaches the skin.

In some embodiments of any one of the embodiments described herein, the ester moiety is terminated by a moiety (represented by variable D) which is relatively bulky, i.e., wherein D comprises at least 3 carbon atoms and/or heteroatoms, optionally at least 4, and optionally at least 5 carbon atoms and/or heteroatoms, optionally such that D is alkyl, alkenyl or alkynyl and B is absent. In some embodiments, the bulky moiety is a non-linear group, comprising for example, a branched moiety (e.g., branched alkyl, alkenyl or alkynyl) and/or a cyclic moiety. In some embodiments, the alkyl, alkenyl or alkynyl group is devoid of an aryl, heteroaryl, heteroalicyclic or cycloalkyl substituent.

In some embodiments, the bulky moiety (represented by variable D) is a cyclic moiety selected from the group consisting of cycloalkyl, heteroalicyclic, aryl and heteroaryl, each being substituted or non-substituted.

As described in International Patent Application Publication WO 2012/140642, ester moieties comprising aryl and heteroaryl tend to exhibit an advantageous half-life in human plasma (e.g., in a range of from 2 to 8 hours), wherein the half-life depends on whether a substituent is present, and on the nature (e.g., size) of the substituent, if present.

For example, compounds comprising a non-substituted aryl (e.g., phenyl) or heteroaryl (e.g., pyrrol-2-yl) moiety exhibit a half-life in human plasma of at least about 2 hours, whereas compounds comprising non-substituted cycloalkyl (e.g., cyclohexyl) exhibit a considerably shorter half-life.

In some embodiments of any one of the embodiments described herein, a substituent of an aryl or heteroaryl is selected from the group consisting of alkyl, alkoxy, aryloxy, hydroxy, amine, nitrile, nitro and halide. In some embodiments, an aryl group is substituted with the aforementioned substituent(s).

As further described in International Patent Application Publication WO 2012/140642, ring substituents (e.g., phenyl substituents) slow hydrolysis considerably, to a degree which is correlated to the size of the substituent.

In some embodiments of any one of the embodiments described herein, the substituent is small, for example, 1 or 2 atoms in size (excluding hydrogen atoms). Examples of such substituents include methyl, ethyl, methoxy, hydroxy, amino (-NH₂), nitrile and halide (fluoro or chloro, in some embodiments). Small substituents may lengthen the half-life of the esterified compound to a significant, but not excessive extent. Thus, for example, compounds having such substituents of 1 or 2 atoms tend to have half-lives in human plasma of up to about 8 hours (480 minutes), whereas larger substituents (e.g., ethoxy) may have considerably longer half-lives (e.g., > 1000 minutes).

In some embodiments of any one of the embodiments described herein, the aryl, heteroaryl, heteroalicyclic or cycloalkyl is attached directly to A, wherein B is absent (e.g., such that the ester moiety is benzoyl or a derivative thereof).

In some embodiments of any one of the embodiments described herein, the aryl, heteroaryl or cycloalkyl is attached via a saturated or unsaturated alkylene chain represented by the variable B (e.g., such that the ester moiety is phenylacetyl or a derivative thereof).

As described in International Patent Application Publication WO 2012/140642, such alkylene chains generally do not exhibit a tendency to slow hydrolysis.

### Miscellaneous definitions:

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthyl and anthracenyl. Phenyl and naphthyl are exemplary aryl groups. The aryl group may be substituted or non-substituted. Exemplary non-substituted aryl groups include non-substituted phenyl and naphthyl. When an aryl is substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, nitrile, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazine, pyrazole, pyridine, pyrimidine, benzopyrone (e.g., 4-oxo-1-benzopyran), quinoline, isoquinoline and purine.

Pyrrole, thiazole, pyrazine and 4-oxo-1-benzopyran are exemplary heteroaryl groups. The heteroaryl group may be substituted or non-substituted. When a heteroaryl is substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, nitrile, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. It is to be appreciated that a substituent (e.g., oxo) may be a component of the conjugated pi-electron system.

A "cycloalkyl" group refers to an all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropyl, cyclobutyl, cyclopentyl, cyclopentenyl, cyclohexyl, cyclohexadiene, cycloheptyl, cycloheptatrienyl, norbornyl (i.e., bicyclo[2.2.1]heptanyl) and adamantyl. Exemplary cycloalkyl groups include cyclopropyl, cyclobutyl, cyclohexyl, norbornyl and adamantyl. A cycloalkyl group may be substituted or non-substituted. Exemplary non-substituted cycloalkyl groups include cyclopropyl, cyclopentyl, cyclohexyl, and adamantyl. When a cycloalkyl is substituted, the substituent group can be, for example, alkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, nitrile, nitro, azide, phosphonyl, phosphinyl, oxo, carbonyl, thiocarbonyl, urea, thiourea, O-carbamyl, N-carbamyl, O-thiocarbamyl, N-thiocarbamyl, C- amido, N-amido, C-carboxy, O-carboxy, sulfonamido, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, nitrile, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. Representative examples are piperidine, piperazine, tetrahydrofuran, tetrahydropyran, morpholine and the like.

As used herein throughout, the term "alkyl" refers to a saturated aliphatic hydrocarbon including straight chain and branched chain groups. Preferably, the alkyl group has 1 to 20 carbon atoms. Whenever a numerical range, e.g., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. When substituted, the substituent group can be, for example, cycloalkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, sulfonate, nitrile, nitro, azide, phosphonyl, phosphinyl, oxo, carbonyl, thiocarbonyl, urea, thiourea, carbamyl, N-carbamyl, O-thiocarbamyl, N-thiocarbamyl, C-amido, N-amido, C-carboxy, O-carboxy, sulfonamido, and amino, as these terms are defined herein.

An "alkenyl" group refers to an unsaturated aliphatic hydrocarbon which comprises at least one carbon-carbon double bond, including straight chain and branched chain groups. Preferably, the alkenyl group has 2 to 20 carbon atoms. More preferably, the alkenyl is a medium size alkenyl having 2 to 10 carbon atoms. Most preferably, unless otherwise indicated, the alkenyl is a lower alkenyl having 2 to 5 carbon atoms. The alkenyl group may be substituted or unsubstituted. When substituted, the substituent group can be, for example, cycloalkyl, alkynyl, aryl, heteroaryl, heteroalicyclic, halo, hydroxy, alkoxy, aryloxy, thiohydroxy, thioalkoxy, thioaryloxy, sulfinyl, sulfonyl, sulfonate, nitrile, nitro, azide, phosphonyl, phosphinyl, oxo, carbonyl, thiocarbonyl, urea, thiourea, carbamyl, N-carbamyl, O-thiocarbamyl, N-thiocarbamyl, C-amido, N-amido, C-carboxy, O-carboxy, sulfonamido, and amino, as these terms are defined herein.

An "alkynyl" group refers to an unsaturated aliphatic hydrocarbon which comprises at least one carbon-carbon triple bond, including straight chain and branched chain groups. Preferably, the alkynyl group has 2 to 20 carbon atoms. More preferably, the alkynyl is a medium size alkynyl having 2 to 10 carbon atoms. Most preferably, unless otherwise indicated, the alkynyl is a lower alkynyl having 2 to 4 carbon atoms. The alkynyl group may be substituted or unsubstituted. When substituted, the substituent group can be, for example, cycloalkyl, alkenyl, aryl, heteroaryl, heteroalicyclic, halo, hydroxy, alkoxy, aryloxy, thiohydroxy, thioalkoxy, thioaryloxy, sulfinyl, sulfonyl, sulfonate, nitrile, nitro, azide, phosphonyl, phosphinyl, oxo, carbonyl, thiocarbonyl, urea, thiourea, carbamyl, N-carbamyl, O-thiocarbamyl, N-thiocarbamyl, C-amido, N-amido, C-carboxy, O-carboxy, sulfonamido, and amino, as these terms are defined herein.

As used herein, the terms "amine" and "amino" refer to a -NR'R" group, wherein R' and R" are selected from the group consisting of hydrogen, alkyl, cycloalkyl, heteroalicyclic (bonded through a ring carbon), aryl and heteroaryl (bonded through a ring carbon). Optionally, R' and R" are selected from the group consisting of hydrogen and alkyl comprising 1 to 4 carbon atoms. Optionally, R' and R" are hydrogen.

A "hydroxy" group refers to an -OH group.
An "azide" group refers to a -N=N⁺=N⁻ group.
An "alkoxy" group refers to both an -O-alkyl and an -O-cycloalkyl group, as defined herein.
An "aryloxy" group refers to both an -O-aryl and an -O-heteroaryl group, as defined herein.
A "thiohydroxy" or "thiol" group refers to a -SH group.
A "thioalkoxy" group refers to both an -S-alkyl group, and an -S-cycloalkyl group, as defined herein.
A "thioaryloxy" group refers to both an -S-aryl and an -S-heteroaryl group, as defined herein.

A "disulfide" group refers to both a -S-thioalkoxy and a -S-thioaryloxy group.

A "carbonyl" group refers to a -C(=O)-R' group, where R' is defined as hereinabove.

A "thiocarbonyl" group refers to a -C(=S)-R' group, where R' is as defined herein.

A "carboxy" group encompasses C-carboxy and O-carboxy groups, as defined herein.

A "C-carboxy" group refers to a -C(=O)-O-R' group, where R' is as defined herein.

An "O-carboxy" group refers to an R'C(=O)-O- group, where R' is as defined herein.

A "carboxylic acid" group refers to a C-carboxy group in which R' is hydrogen.

A "thiocarboxy" or "thiocarboxylate" group refers to both -C(=S)-O-R' and -O-C(=S)R' groups.

A "sulfonate" group refers to both -S(=O)₂-O-R' and -O-S(=O)₂-R' groups, where R' is as defined herein.

An "ester" refers herein to both carboxy esters and sulfonate esters.

A "carboxy ester" refers to an O-carboxy group attached to a carbon atom.

A "sulfonate ester" refers to a -O-S(=O)₂-R' sulfonate group attached to a carbon atom.

A "halide" or "halo" group refers to fluorine, chlorine, bromine or iodine.

A "sulfinyl" group refers to an -S(=O)-R' group, where R' is as defined herein.

A "sulfonyl" group refers to an -S(=O)₂-R' group, where R' is as defined herein.

A "sulfate" group refers to an -O-S(=O)₂-O-R' group, where R' is as defined as herein.

A "sulfonamide" or "sulfonamido" group encompasses both S-sulfonamido and N-sulfonamido groups, as defined herein.

An "S-sulfonamido" group refers to a -S(=O)₂-NR'R" group, with each of R' and R" as defined herein.

An "N-sulfonamido" group refers to an R'S(=O)₂-NR" group, where each of R' and R" is as defined herein.

An "O-carbamyl" group refers to an -OC(=O)-NR'R" group, where each of R' and R" is as defined herein.

An "N-carbamyl" group refers to an R'OC(=O)-NR"- group, where each of R' and R" is as defined herein.

A "carbamyl" or "carbamate" group encompasses O-carbamyl and N-carbamyl groups.

An "O-thiocarbamyl"" group refers to an -OC(=S)-NR'R" group, where each of R' and R" is as defined herein.

An "N-thiocarbamyl" group refers to an R'OC(=S)NR"- group, where each of R' and R" is as defined herein.

A "thiocarbamyl" or "thiocarbamate" group encompasses O-thiocarbamyl and N-thiocarbamyl groups.

A thiocarbamate bond describes a -O-C(=S)-NR'- bond, where R' is as described herein.

A "C-amido" group refers to a -C(=O)-NR'R" group, where each of R' and R" is as defined herein.

An "N-amido" group refers to an R'C(=O)-NR"- group, where each of R' and R" is as defined herein.

An "amide" group encompasses both C-amido and N-amido groups.

A "nitro" group refers to an -NO₂ group.

A "nitroso" group refers to an -NO group.

A "nitrile" or "cyano" group refers to a -C≡N group.

An "isonitrile" group refers to a -N≡C group.

An "oxo" group refers to a =O group.

The term "phosphonyl" or "phosphonate" describes a -P(=O)(OR')(OR") group, with R' and R" as defined hereinabove.

The term "phosphate" describes an -O-P(=O)(OR')(OR") group, with each of R' and R" as defined hereinabove.

The term "phosphinyl" describes a -PR'R" group, with each of R' and R" as defined hereinabove.

A "urea" group refers to an -N(R')-C(=O)-NR"R‴ group, where each of R' and R" is as defined herein, and R‴ is defined as R' and R" are defined herein.

The term "thiourea" describes a -N(R')-C(=S)-NR"- group, with each of R' and R" as defined hereinabove.

As used herein, the term "epoxide" describes a 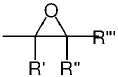 group, where R', R" and R‴ are as defined herein.

As used herein, the term "thiirane" describes a group that is equivalent to an epoxide, wherein the oxygen atom of the epoxide is replaced with a sulfur atom.

As used herein, the term "aziridine" describes a group that is equivalent to an epoxide, wherein the oxygen atom of the epoxide is replaced with a nitrogen atom, and the nitrogen atom binds, in addition to two adjacent carbon atoms, Rʺʺ, wherein Rʺʺ is defined according to the same definition as R'.

The term "hydrazine", as used herein, describes a -NR'-NR"R‴ group, with R', R" and R‴ as defined herein.

As used herein the terms "treating", "treatment" and any grammatical diversion thereof include abrogating, substantially inhibiting, slowing or reversing the progression of a condition, substantially ameliorating clinical or aesthetical symptoms of a condition or substantially preventing the appearance of clinical or aesthetical symptoms of a condition. The condition may be a cosmetic condition and/or a medical condition.

As used herein the term "about" refers to ± 20 %. In some embodiments of any of the respective embodiments described herein, the term "about" is to be interpreted as ± 10%.

The terms "comprises", "comprising", "includes", "including", "having" and their conjugates mean "including but not limited to".

The term "consisting of" means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### MATERIALS AND METHODS

### Materials:

Carbopol^{®} Ultrez 10 (cross-linked polyacrylic acid) was obtained from Noveon Inc. (USA);
Gelucire^{®} 44/14 (lauroyl polyoxyl-32 glycerides) was obtained from Gattefosse (France);
Gelucire^{®} 50/13 (macrogolglycerol stearates) was obtained from Gattefosse;
Labrasol^{®} (caprylocaproyl polyoxyl-8 glycerides) was obtained from Gattefosse;
Lauroglycol^{™} 90 (propylene glycol monolaurate) was obtained from Gattefosse;
Phosphatidyl choline was obtained from Lipoid (Germany);
Propylene glycol was obtained from MP Biomedical (France);
Vitamin E TPGS (α-tocopheryl polyethylene glycol succinate) was obtained from Eastman (UK);
R-55 (2-((4-(3-hydroxyphenyl)piperazin-1-yl)methyl)quinazolin-4-one) was synthesized as described in International Patent Application Publication WO 2012/140642.

### R-55 formulation for topical application and effect on skin:

A formulation for topical administration of R-55 (2-((4-(3-hydroxyphenyl)piperazin-1-yl)methyl)quinazolin-4-one) was prepared, comprising 10 mg/ml R-55.

A lipid-based phase was formed by mixing 60 grams propylene glycol (a solvent of R-55 and penetration enhancer), 1 gram ethanol, 15 grams phosphatidyl choline (a surfactant), 10 grams Gelucire^{®} 44/14 lauroyl macrogolglycerides (a solubilizer of R-55), 5 grams Gelucire^{®} 50/13 stearoyl macrogolglycerides (a sustained release agent), 1 gram Labrasol^{®} caprylocaproyl macrogolglycerides (a co-surfactant and penetration enhancer), 1 gram Lauroglycol^{™} 90 (a solubilizer of R-55, co-surfactant and penetration enhancer), and 0.5 gram vitamin E TPGS (a stabilizer), along with 1 gram R-55. The optimal ratio of propylene glycol to phosphatidyl choline (with respect to long-term dissolution of R-55) was determined to be about 4:1.

At first, the propylene glycol and phosphatidyl choline were mixed by stirring at a temperature in a range of from 40 °C to 60 °C until the phosphatidyl choline was completely dissolved (for 1-2 hours), as indicated by obtaining a clear solution. The R-55, ethanol, Labrasol^{®} caprylocaproyl macrogolglycerides, Lauroglycol^{™} 90, Gelucire^{®} 44/14 lauroyl macrogolglycerides and Gelucire^{®} 50/13 stearoyl macrogolglycerides were then added, followed by stirring at 40-60 °C for 2-4 hours (in order to dissolve the R-55), to complete the lipid-based phase.

After being cooled to room temperature, the lipid-based phase with R-55 was added to 5.5 grams of a 0.4 % aqueous solution of a carbomer (Carbopol^{®} Ultrez 10) to obtain 100 grams of a suspension, which was stirred continuously at room temperature for approximately 2 hours. A gel-like water-in-oil emulsion was formed, with relatively round aqueous droplets dispersed in a lipophilic phase, and the emulsion was stable for at least 2 weeks at room temperature.

The Carbopol^{®} Ultrez 10 carbomer was found to provide a particularly pleasant feel when used in the aqueous phase, in comparison with Carbopol^{®} 974PNF, Carbopol^{®} Ultrez 20 and Carbopol^{®} Ultrez 21 and carboxymethylcellulose.

Two middle-aged Caucasian female volunteers self-administered R-55 by applying daily the topical formulation of R-55 at discrete locations on the face and behind the ear.

No red marks were observed on the skin after application, suggesting that the formulation does not significantly enhance local blood flow and is not inflammatory.

After about one month of daily usage, the skin of the subjects was noticeably less pigmented, and had an overall fresher, younger appearance. This effect was not limited to skin upon which the formulation was applied, indicating that intradermal and/or transdermal administration of the compound was effected.

### Safety and toxicology studies:

A formulation for topical administration of R-55 (10 mg/ml), prepared as described hereinabove, was tested for the presence of substances prohibited from inclusion in cosmetic products, including cadmium, mercury and lead, as well as for the presence of microbiological contaminants such as *Staphylococcus aureus, Pseudomonas aeruginosa* and *Candida* yeast, in an accredited laboratory (via International Laboratory Accreditation Cooperation (ILAC) Mutual Recognition Agreement). The tests confirmed the absence of prohibited substances as wells as microbiological contamination.

Safety and toxicology is than evaluated by applying the formulation to human volunteers in a controlled study, in accordance with European Union standards. To the extent that section headings are used, they should not be construed as necessarily limiting.

## Claims

1. A method of improving the appearance of aging skin in a subject in need thereof, the method comprising topically administering a composition comprising a compound of the general Formula I: 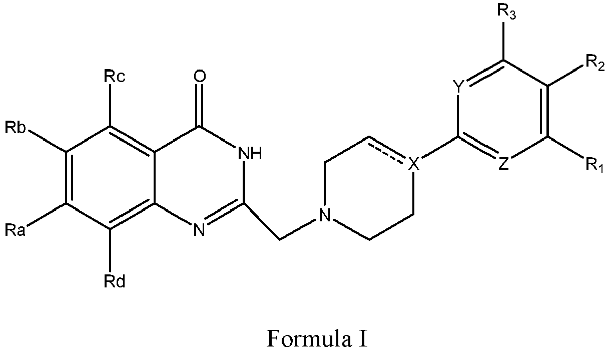
or a pharmaceutically acceptable salt thereof,
wherein:
the dashed line denotes a saturated or non-saturated bond;
X is selected from the group consisting of CH, C and N, such that when X is C the dashed line denotes a non-saturated bond and when X is CH or N the dashed line denotes a saturated bond;
Y is N or CR₄;
Z is N or CR₅; and
Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
and a carrier suitable for topical administration on skin, being in a form of a water-in-oil emulsion, said emulsion comprising:
from 30 to 80 weight percents propylene glycol;
from 7.5 to 30 weight percents phosphatidyl choline;
from 5 to 20 weight percents lauroyl macrogolglycerides;
from 2.5 to 10 weight percents macrogolglycerol stearate;
from 0 to 2 weight percents ethanol;
from 0 to 2 weight percents caprylocaproyl macrogolglycerides;
from 0 to 2 weight percents propylene glycol monolaurate; and
from 2 to 20 weight percents water.

2. A non-therapeutic method of lightening skin in a subject in need thereof, the method comprising topically administering a composition comprising a compound of the general Formula I: 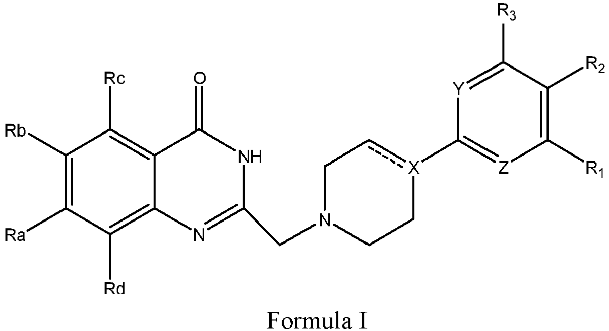
or a pharmaceutically acceptable salt thereof,
wherein:
the dashed line denotes a saturated or non-saturated bond;
X is selected from the group consisting of CH, C and N, such that when X is C the dashed line denotes a non-saturated bond and when X is CH or N the dashed line denotes a saturated bond;
Y is N or CR₄;
Z is N or CR₅; and
Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
and a carrier suitable for topical administration on skin, being in a form of a water-in-oil emulsion, said emulsion comprising:
from 30 to 80 weight percents propylene glycol;
from 7.5 to 30 weight percents phosphatidyl choline;
from 5 to 20 weight percents lauroyl macrogolglycerides;
from 2.5 to 10 weight percents macrogolglycerol stearate;
from 0 to 2 weight percents ethanol;
from 0 to 2 weight percents caprylocaproyl macrogolglycerides;
from 0 to 2 weight percents propylene glycol monolaurate; and
from 2 to 20 weight percents water.

3. A non-therapeutic method of treating uneven skin pigmentation in a subject in need thereof, the method comprising topically administering to the subject a composition comprising a compound of the general Formula I: 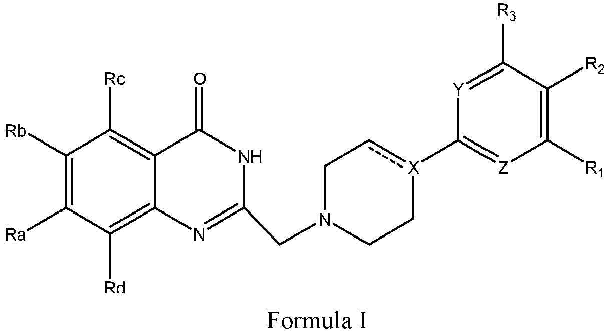
or a pharmaceutically acceptable salt thereof,
wherein:
the dashed line denotes a saturated or non-saturated bond;
X is selected from the group consisting of CH, C and N, such that when X is C the dashed line denotes a non-saturated bond and when X is CH or N the dashed line denotes a saturated bond;
Y is N or CR₄;
Z is N or CR₅; and
Ra-Rd, and R₁-R₅ are each independently selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted,
and a carrier suitable for topical administration on skin, being in a form of a water-in-oil emulsion, said emulsion comprising:
from 30 to 80 weight percents propylene glycol;
from 7.5 to 30 weight percents phosphatidyl choline;
from 5 to 20 weight percents lauroyl macrogolglycerides;
from 2.5 to 10 weight percents macrogolglycerol stearate;
from 0 to 2 weight percents ethanol;
from 0 to 2 weight percents caprylocaproyl macrogolglycerides;
from 0 to 2 weight percents propylene glycol monolaurate; and
from 2 to 20 weight percents water.

4. The method of any one of claims 1 to 3, wherein a concentration of propylene glycol in said composition is about 60 weight percents.

5. The method of any one of claims 1 to 4, wherein a concentration of phosphatidyl choline in said composition is about 15 weight percents.

6. The method of any one of claims 1 to 5, wherein a concentration of lauroyl macrogolglycerides in said composition is about 10 weight percents.

7. The method of any one of claims 1 to 6, wherein a concentration of macrogolglycerol stearate in said composition is about 5 weight percents.

8. The method of any one of claims 1 to 7, wherein a concentration of water in said composition is about 5.5 weight percents.

9. The method of any one of claims 1 to 8, wherein a concentration of the compound of said general Formula I in the composition is at least 5 mg/ml.

10. The method of any one of claims 1 to 9, wherein a weight ratio of propylene glycol to phosphatidyl choline in said composition is in range of 3:1 to 5:1.

11. The method of any one of claims 1 to 10, wherein an aqueous phase of said emulsion comprises an aqueous solution of a carbomer.

12. The method of any one of claims 1 to 11, wherein at least one of R₁-R₅ is selected from the group consisting of hydroxy and a moiety having the general Formula II: 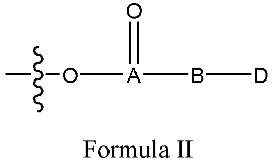 wherein:
A is selected from the group consisting of C and S=O;
B is absent or is a substituted or non-substituted, saturated or non-saturated alkylene chain; and
D is selected from the group consisting of hydrogen, alkyl, alkenyl, alkynyl, cycloalkyl, heteroalicyclic, aryl, heteroaryl, hydroxy, alkoxy, aryloxy, thiohydroxy, thioalkoxy, thioaryloxy, halide, amine, amide, carbonyl, thiocarbonyl, carboxy, thiocarboxy, epoxide, sulfonate, sulfonyl, sulfinyl, sulfonamide, nitro, nitrile, isonitrile, thiirane, aziridine, nitroso, hydrazine, sulfate, azide, phosphonyl, phosphinyl, urea, thiourea, carbamyl and thiocarbamyl, each being substituted or non-substituted.

13. The method of any one of claims 1 to 11, wherein said compound is 2-((4-(3-hydroxyphenyl)piperazin-1-yl)methyl)quinazolin-4-one:

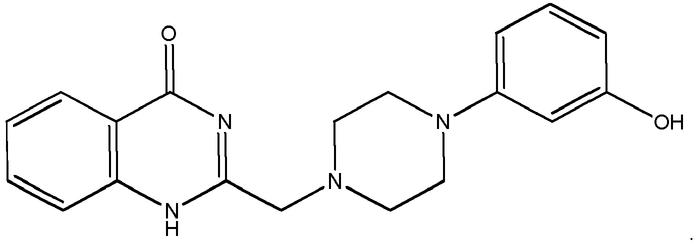

## Patentansprüche

1. Verfahren zum Verbessern des Aussehens von alternder Haut bei einem Individuum, welches dessen bedarf, wobei das Verfahren ein topisches Verabreichen einer Zusammensetzung umfasst, die eine Verbindung der allgemeinen Formel I: 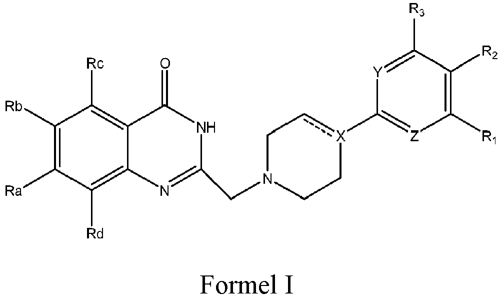
oder ein pharmazeutisch unbedenkliches Salz derselben umfasst,
wobei:
die gestrichelte Linie eine gesättigte oder ungesättigte Bindung bezeichnet,
X aus der Gruppe ausgewählt ist, die aus CH, C und N besteht, derart, dass die gestrichelte Linie eine ungesättigte Bindung bezeichnet, wenn X für C steht, und die gestrichelte Linie eine gesättigte Bindung bezeichnet, wenn X für CH oder N steht;
Y für N oder CR₄ steht;
Z für N oder CR₅ steht; und
Ra bis Rd und R₁ bis R₅ jeweils auf unabhängige Weise aus der Gruppe ausgewählt sind, die aus Wasserstoff, Alkyl, Alkenyl, Alkinyl, Cycloalkyl, heteroalizyklischen Bausteinen, Aryl, Heteroaryl, Hydroxyl, Alkoxy, Aryloxy, Thiohydroxy, Thioalkoxy, Thioaryloxy, Halogenid, Amin, Amid, Carbonyl, Thiocarbonyl, Carboxy, Thiocarboxy, Epoxid, Sulfonat, Sulfonyl, Sulfinyl, Sulfonamid, Nitro, Nitril, Isonitril, Thiiran, Aziridin, Nitroso, Hydrazin, Sulfat, Azid, Phosphonyl, Phosphinyl, Harnstoff, Thioharnstoff, Carbamyl und Thiocarbamyl besteht, wobei diese jeweils substituiert oder unsubsituiert vorliegen können,
und einen Trägerstoff, der sich zur topischen Verabreichung an die Haut eignet, wobei er in Form einer Wasser-in-Öl-Emulsion vorliegt, wobei die Emulsion Folgendes umfasst:
30 bis 80 Gewichtsprozent an Propylenglykol;
7,5 bis 30 Gewichtsprozent an Phosphatidylcholin;
5 bis 20 Gewichtsprozent an Lauroylmakrogolglyceriden;
2,5 bis 10 Gewichtsprozent an Makrogolglycerinstearat;
0 bis 2 Gewichtsprozent an Ethanol;
0 bis 2 Gewichtsprozent an Caprylocaproylmakrogolglyceriden;
0 bis 2 Gewichtsprozent an Propylenglykolmonolaurat; und
2 bis 20 Gewichtsprozent an Wasser.

2. Nicht-therapeutisches Verfahren zum Aufhellen der Haut bei einem Individuum, welches dessen bedarf, wobei das Verfahren ein topisches Verabreichen einer Zusammensetzung umfasst, die eine Verbindung der allgemeinen Formel I: 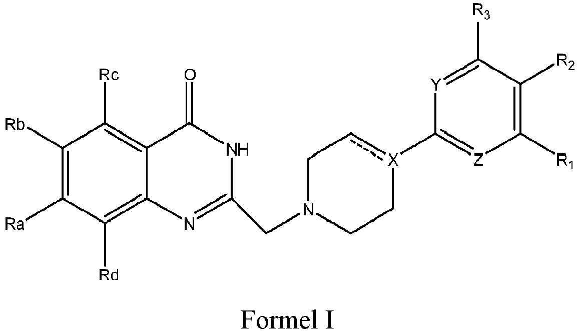
oder ein pharmazeutisch unbedenkliches Salz derselben umfasst,
wobei:
die gestrichelte Linie eine gesättigte oder ungesättigte Bindung bezeichnet,
X aus der Gruppe ausgewählt ist, die aus CH, C und N besteht, derart, dass die gestrichelte Linie eine ungesättigte Bindung bezeichnet, wenn X für C steht, und die gestrichelte Linie eine gesättigte Bindung bezeichnet, wenn X für CH oder N steht;
Y für N oder CR₄ steht;
Z für N oder CR₅ steht; und
Ra bis Rd und R₁ bis R₅ jeweils auf unabhängige Weise aus der Gruppe ausgewählt sind, die aus Wasserstoff, Alkyl, Alkenyl, Alkinyl, Cycloalkyl, heteroalizyklischen Bausteinen, Aryl, Heteroaryl, Hydroxyl, Alkoxy, Aryloxy, Thiohydroxy, Thioalkoxy, Thioaryloxy, Halogenid, Amin, Amid, Carbonyl, Thiocarbonyl, Carboxy, Thiocarboxy, Epoxid, Sulfonat, Sulfonyl, Sulfinyl, Sulfonamid, Nitro, Nitril, Isonitril, Thiiran, Aziridin, Nitroso, Hydrazin, Sulfat, Azid, Phosphonyl, Phosphinyl, Harnstoff, Thioharnstoff, Carbamyl und Thiocarbamyl besteht, wobei diese jeweils substituiert oder unsubsituiert vorliegen können,
und einen Trägerstoff, der sich zur topischen Verabreichung an die Haut eignet, wobei er in Form einer Wasser-in-Öl-Emulsion vorliegt, wobei die Emulsion Folgendes umfasst:
30 bis 80 Gewichtsprozent an Propylenglykol;
7,5 bis 30 Gewichtsprozent an Phosphatidylcholin;
5 bis 20 Gewichtsprozent an Lauroylmakrogolglyceriden;
2,5 bis 10 Gewichtsprozent an Makrogolglycerinstearat;
0 bis 2 Gewichtsprozent an Ethanol;
0 bis 2 Gewichtsprozent an Caprylocaproylmakrogolglyceriden;
0 bis 2 Gewichtsprozent an Propylenglykolmonolaurat; und
2 bis 20 Gewichtsprozent an Wasser.

3. Nicht-therapeutisches Verfahren zur Behandlung ungleichmäßiger Hautpigmentierung bei einem Individuum, welches dessen bedarf, wobei das Verfahren ein topisches Verabreichen einer Zusammensetzung umfasst, die eine Verbindung der allgemeinen Formel I: 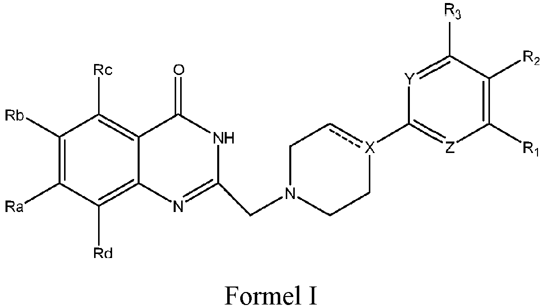
oder ein pharmazeutisch unbedenkliches Salz derselben umfasst,
wobei:
die gestrichelte Linie eine gesättigte oder ungesättigte Bindung bezeichnet,
X aus der Gruppe ausgewählt ist, die aus CH, C und N besteht, derart, dass die gestrichelte Linie eine ungesättigte Bindung bezeichnet, wenn X für C steht, und die gestrichelte Linie eine gesättigte Bindung bezeichnet, wenn X für CH oder N steht;
Y für N oder CR₄ steht;
Z für N oder CR₅ steht; und
Ra bis Rd und R₁ bis R₅ jeweils auf unabhängige Weise aus der Gruppe ausgewählt sind, die aus Wasserstoff, Alkyl, Alkenyl, Alkinyl, Cycloalkyl, heteroalizyklischen Bausteinen, Aryl, Heteroaryl, Hydroxyl, Alkoxy, Aryloxy, Thiohydroxy, Thioalkoxy, Thioaryloxy, Halogenid, Amin, Amid, Carbonyl, Thiocarbonyl, Carboxy, Thiocarboxy, Epoxid, Sulfonat, Sulfonyl, Sulfinyl, Sulfonamid, Nitro, Nitril, Isonitril, Thiiran, Aziridin, Nitroso, Hydrazin, Sulfat, Azid, Phosphonyl, Phosphinyl, Harnstoff, Thioharnstoff, Carbamyl und Thiocarbamyl besteht, wobei diese jeweils substituiert oder unsubsituiert vorliegen können,
und einen Trägerstoff, der sich zur topischen Verabreichung an die Haut eignet, wobei er in Form einer Wasser-in-Öl-Emulsion vorliegt, wobei die Emulsion Folgendes umfasst:
30 bis 80 Gewichtsprozent an Propylenglykol;
7,5 bis 30 Gewichtsprozent an Phosphatidylcholin;
5 bis 20 Gewichtsprozent an Lauroylmakrogolglyceriden;
2,5 bis 10 Gewichtsprozent an Makrogolglycerinstearat;
0 bis 2 Gewichtsprozent an Ethanol;
0 bis 2 Gewichtsprozent an Caprylocaproylmakrogolglyceriden;
0 bis 2 Gewichtsprozent an Propylenglykolmonolaurat; und
2 bis 20 Gewichtsprozent an Wasser.

4. Verfahren nach einem beliebigen der Ansprüche 1 bis 3, wobei eine Konzentration an Propylenglykol in der Zusammensetzung ungefähr 60 Gewichtsprozent beträgt.

5. Verfahren nach einem beliebigen der Ansprüche 1 bis 4, wobei eine Konzentration an Phosphatidylcholin in der Zusammensetzung ungefähr 15 Gewichtsprozent beträgt.

6. Verfahren nach einem beliebigen der Ansprüche 1 bis 5, wobei eine Konzentration an Lauroylmakrogolglyceriden in der Zusammensetzung ungefähr 10 Gewichtsprozent beträgt.

7. Verfahren nach einem beliebigen der Ansprüche 1 bis 6, wobei eine Konzentration an Makrogolglycerinstearat in der Zusammensetzung ungefähr 5 Gewichtsprozent beträgt.

8. Verfahren nach einem beliebigen der Ansprüche 1 bis 7, wobei eine Konzentration an Wasser in der Zusammensetzung ungefähr 5,5 Gewichtsprozent beträgt.

9. Verfahren nach einem beliebigen der Ansprüche 1 bis 8, wobei eine Konzentration der Verbindung der allgemeinen Formel I in der Zusammensetzung mindestens 5 mg/mL beträgt.

10. Verfahren nach einem beliebigen der Ansprüche 1 bis 9, wobei das Gewichtsverhältnis von Propylenglykol zu Phosphatidylcholin in der Zusammensetzung im Bereich von 3:1 bis 5:1 liegt.

11. Verfahren nach einem beliebigen der Ansprüche 1 bis 10, wobei eine wässrige Phase der Emulsion eine wässrige Lösung eines Carbomers umfasst.

12. Verfahren nach einem beliebigen der Ansprüche 1 bis 11, wobei mindestens eines von R₁ bis R₅ aus der Gruppe ausgewählt ist, die aus einem Hydroxyl und einem Baustein besteht, welcher die allgemeine Formel II hat: 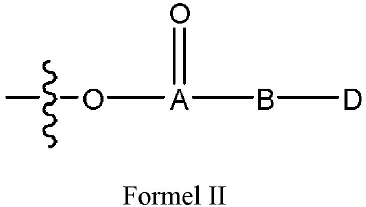 wobei:
A aus der Gruppe ausgewählt ist, die aus C und S=O besteht;
B fehlt oder es sich um eine substituierte oder unsubstutuierte Alkylenkette gesättigter oder ungesättigter Art handelt; und
D aus der Gruppe ausgewählt ist, die aus Wasserstoff, Alkyl, Alkenyl, Alkinyl, Cycloalkyl, heteroalizyklischen Bausteinen, Aryl, Heteroaryl, Hydroxyl, Alkoxy, Aryloxy, Thiohydroxy, Thioalkoxy, Thioaryloxy, Halogenid, Amin, Amid, Carbonyl, Thiocarbonyl, Carboxy, Thiocarboxy, Epoxid, Sulfonat, Sulfonyl, Sulfinyl, Sulfonamid, Nitro, Nitril, Isonitril, Thiiran, Aziridin, Nitroso, Hydrazin, Sulfat, Azid, Phosphonyl, Phosphinyl, Harnstoff, Thioharnstoff, Carbamyl und Thiocarbamyl besteht, wobei diese jeweils substituiert oder unsubsituiert vorliegen können.

13. Verfahren nach einem beliebigen der Ansprüche 1 bis 11, wobei es sich bei der Verbindung um 2-((4-(3-Hydroxyphenyl)piperazin-1-yl)methyl)chinazolin-4-on handelt:

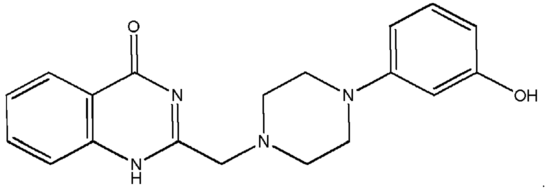

## Revendications

1. Procédé d'amélioration de l'apparence d'une peau vieillissante chez un sujet qui en a besoin, le procédé comprenant l'administration topique d'une composition comprenant un composé de la Formule I générale : 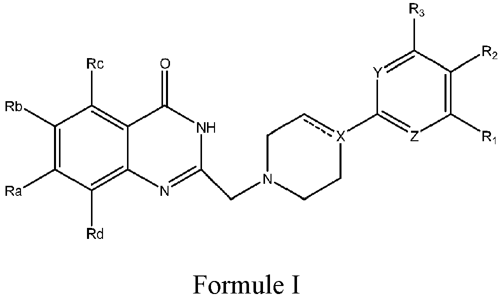
ou un sel pharmaceutiquement acceptable de celui-ci,
dans lequel :
la ligne en pointillés indique une liaison saturée ou non saturée ;
X est sélectionné dans le groupe constitué de CH, C et N, de sorte que lorsque X est C, la ligne en pointillés indique une liaison non saturée et lorsque X est CH ou N, la ligne en pointillés indique une liaison saturée ;
Y est N ou CR₄ ;
Z est N ou CR₅ ; et
Ra-Rd et R₁-R₅ sont chacun sélectionnés indépendamment dans le groupe constitué d'hydrogène, d'alkyle, d'alcényle, d'alcynyle, de cycloalkyle, d'hétéroalicyclique, d'aryle, d'hétéroaryle, d'hydroxy, d'alcoxy, d'aryloxy, de thiohydroxy, de thioalcoxy, de thioaryloxy, d'halogénure, d'amine, d'amide, de carbonyle, de thiocarbonyle, de carboxy, de thiocarboxy, d'époxyde, de sulfonate, de sulfonyle, de sulfinyle, de sulfonamide, de nitro, de nitrile, d'isonitrile, de thiirane, d'aziridine, de nitroso, d'hydrazine, de sulfate, d'azoture, de phosphonyle, de phosphinyle, d'urée, de thiourée, de carbamyle et de thiocarbamyle, chacun étant substitué ou non substitué,
et un support adapté à une administration topique sur la peau, se présentant sous la forme d'une émulsion eau-dans-huile, ladite émulsion comprenant :
de 30 à 80 pour cent en poids de propylène glycol ;
de 7,5 à 30 pour cent en poids de phosphatidylcholine ;
de 5 à 20 pour cent en poids de macrogolglycérides de lauroyle ;
de 2,5 à 10 pour cent en poids de stéarate de macrogolglycérol ;
de 0 à 2 pour cent en poids d'éthanol ;
de 0 à 2 pour cent en poids de macrogolglycérides de caprylocaproyle ;
de 0 à 2 pour cent en poids de monolaurate de propylène glycol ; et
de 2 à 20 pour cent en poids d'eau.

2. Procédé non thérapeutique d'éclaircissement de la peau d'un sujet qui en a besoin, le procédé comprenant l'administration topique d'une composition comprenant un composé de la Formule I générale : 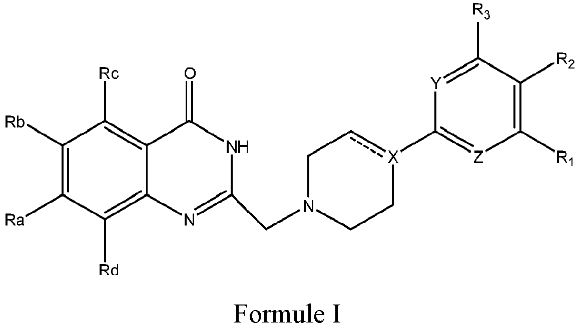
ou un sel pharmaceutiquement acceptable de celui-ci,
dans lequel :
la ligne en pointillés indique une liaison saturée ou non saturée ;
X est sélectionné dans le groupe constitué de CH, C et N, de sorte que lorsque X est C, la ligne en pointillés indique une liaison non saturée et lorsque X est CH ou N, la ligne en pointillés indique une liaison saturée ;
Y est N ou CR₄ ;
Z est N ou CR₅ ; et
Ra-Rd et R₁-R₅ sont chacun sélectionnés indépendamment dans le groupe constitué d'hydrogène, d'alkyle, d'alcényle, d'alcynyle, de cycloalkyle, d'hétéroalicyclique, d'aryle, d'hétéroaryle, d'hydroxy, d'alcoxy, d'aryloxy, de thiohydroxy, de thioalcoxy, de thioaryloxy, d'halogénure, d'amine, d'amide, de carbonyle, de thiocarbonyle, de carboxy, de thiocarboxy, d'époxyde, de sulfonate, de sulfonyle, de sulfinyle, de sulfonamide, de nitro, de nitrile, d'isonitrile, de thiirane, d'aziridine, de nitroso, d'hydrazine, de sulfate, d'azoture, de phosphonyle, de phosphinyle, d'urée, de thiourée, de carbamyle et de thiocarbamyle, chacun étant substitué ou non substitué,
et un support adapté à une administration topique sur la peau, se présentant sous la forme d'une émulsion eau-dans-huile, ladite émulsion comprenant :
de 30 à 80 pour cent en poids de propylène glycol ;
de 7,5 à 30 pour cent en poids de phosphatidylcholine ;
de 5 à 20 pour cent en poids de macrogolglycérides de lauroyle ;
de 2,5 à 10 pour cent en poids de stéarate de macrogolglycérol ;
de 0 à 2 pour cent en poids d'éthanol ;
de 0 à 2 pour cent en poids de macrogolglycérides de caprylocaproyle ;
de 0 à 2 pour cent en poids de monolaurate de propylène glycol ; et
de 2 à 20 pour cent en poids d'eau.

3. Procédé non thérapeutique de traitement d'une pigmentation cutanée irrégulière chez un sujet qui en a besoin, le procédé comprenant l'administration topique au sujet d'une composition comprenant un composé de la Formule I générale : 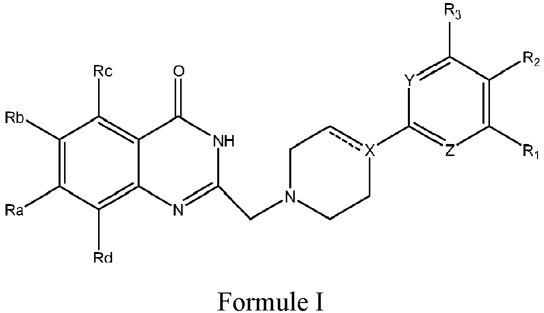
ou un sel pharmaceutiquement acceptable de celui-ci,
dans lequel :
la ligne en pointillés indique une liaison saturée ou non saturée ;
X est sélectionné dans le groupe constitué de CH, C et N, de sorte que lorsque X est C, la ligne en pointillés indique une liaison non saturée et lorsque X est CH ou N, la ligne en pointillés indique une liaison saturée ;
Y est N ou CR₄ ;
Z est N ou CR₅ ; et
Ra-Rd et R₁-R₅ sont chacun sélectionnés indépendamment dans le groupe constitué d'hydrogène, d'alkyle, d'alcényle, d'alcynyle, de cycloalkyle, d'hétéroalicyclique, d'aryle, d'hétéroaryle, d'hydroxy, d'alcoxy, d'aryloxy, de thiohydroxy, de thioalcoxy, de thioaryloxy, d'halogénure, d'amine, d'amide, de carbonyle, de thiocarbonyle, de carboxy, de thiocarboxy, d'époxyde, de sulfonate, de sulfonyle, de sulfinyle, de sulfonamide, de nitro, de nitrile, d'isonitrile, de thiirane, d'aziridine, de nitroso, d'hydrazine, de sulfate, d'azoture, de phosphonyle, de phosphinyle, d'urée, de thiourée, de carbamyle et de thiocarbamyle, chacun étant substitué ou non substitué,
et un support adapté à une administration topique sur la peau, se présentant sous la forme d'une émulsion eau-dans-huile, ladite émulsion comprenant :
de 30 à 80 pour cent en poids de propylène glycol ;
de 7,5 à 30 pour cent en poids de phosphatidylcholine ;
de 5 à 20 pour cent en poids de macrogolglycérides de lauroyle ;
de 2,5 à 10 pour cent en poids de stéarate de macrogolglycérol ;
de 0 à 2 pour cent en poids d'éthanol ;
de 0 à 2 pour cent en poids de macrogolglycérides de caprylocaproyle ;
de 0 à 2 pour cent en poids de monolaurate de propylène glycol ; et
de 2 à 20 pour cent en poids d'eau.

4. Procédé selon l'une quelconque des revendications 1 à 3, dans lequel une concentration en propylène glycol dans ladite composition est d'environ 60 pour cent en poids.

5. Procédé selon l'une quelconque des revendications 1 à 4, dans lequel une concentration en phosphatidylcholine dans ladite composition est d'environ 15 pour cent en poids.

6. Procédé selon l'une quelconque des revendications 1 à 5, dans lequel une concentration de macrogolglycérides de lauroyle dans ladite composition est d'environ 10 pour cent en poids.

7. Procédé selon l'une quelconque des revendications 1 à 6, dans lequel une concentration en stéarate de macrogolglycérol dans ladite composition est d'environ 5 pour cent en poids.

8. Procédé selon l'une quelconque des revendications 1 à 7, dans lequel une concentration en eau dans ladite composition est d'environ 5,5 pour cent en poids.

9. Procédé selon l'une quelconque des revendications 1 à 8, dans lequel une concentration en composé de ladite Formule I générale dans la composition est d'au moins 5 mg/ml.

10. Procédé selon l'une quelconque des revendications 1 à 9, dans lequel un rapport pondéral entre le propylène glycol et la phosphatidylcholine dans ladite composition est dans la plage de 3:1 à 5:1.

11. Procédé selon l'une quelconque des revendications 1 à 10, dans lequel une phase aqueuse de ladite émulsion comprend une solution aqueuse d'un carbomère.

12. Procédé selon l'une quelconque des revendications 1 à 11, dans lequel au moins l'un des R₁-R₅ est sélectionné dans le groupe constitué d'hydroxy et d'une fraction ayant la Formule II générale : 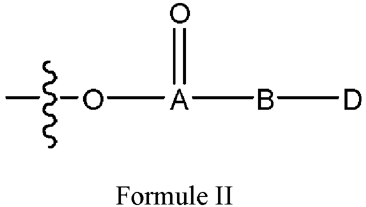 dans lequel :
A est sélectionné dans le groupe constitué de C et S=O ;
B est absent ou est une chaîne alkylène substituée ou non substituée, saturée ou non saturée ; et
D est choisi dans le groupe constitué d'hydrogène, d'alkyle, d'alcényle, d'alcynyle, de cycloalkyle, d'hétéroalicyclique, d'aryle, d'hétéroaryle, d'hydroxy, d'alcoxy, d'aryloxy, de thiohydroxy, de thioalcoxy, de thioaryloxy, d'halogénure, d'amine, d'amide, de carbonyle, de thiocarbonyle, de carboxy, de thiocarboxy, d'époxyde, de sulfonate, de sulfonyle, de sulfinyle, de sulfonamide, de nitro, de nitrile, d'isonitrile, de thiirane, d'aziridine, de nitroso, d'hydrazine, de sulfate, d'azoture, de phosphonyle, de phosphinyle, d'urée, de thiourée, de carbamyle et de thiocarbamyle, chacun étant substitué ou non substitué.

13. Procédé selon l'une quelconque des revendications 1 à 11, dans lequel ledit composé est 2-((4-(3-hydroxyphényl)pipérazine-1-yl)méthyl)quinazolin-4-one :
